# EUROPEAN PATENT APPLICATION

(11) **EP 3 812 768 A1**
(43) Date of publication of application: **28.04.2021**
(21) Application number: 19853623.7
(22) Date of filing: 30.08.2019
(51) Int. Cl.: G01N 33/569, A61K 39/008

(54) **METHOD FOR DETECTING ANTIBODIES AGAINST LEISHMANIA IN A CANINE BIOLOGICAL SAMPLE, KIT FOR DETECTION OF LEISHMANIA INFECTION IN A BIOLOGICAL SAMPLE, AND USE OF LEISHMANIA LIPOPHOSPHOGLUCAN**

(30) Priority: 31.08.2018 BR 102018067339
(71) Applicant: Fundação Oswaldo Cruz, 21045-900 Rio de Janeiro, RJ (BR)
(72) Inventor: SOARES, Rodrigo Pedro Pinto, 30320-080 Belo Horizonte, MG (BR); PORTELA, Ricardo Wagner Dias, 40140-100 Salvador, BA (BR); MELO, Stella Maria Barrouin, 40100-060 Salvador, BA (BR); PASSOS, Gabriela Porfírio, 41650-320 Salvador, BA (BR); BARRAL, Thiago Assis Doria, 41750-166 Salvador, BA (BR)
(74) Representative: Weidner Stern Jeschke
(86) International application number: PCT/BR2019/050356
(87) International publication number: WO 2020/041849

(57) **Abstract**

Visceral leishmaniasis is one of the most neglected diseases in the world and affects humans and other mammals. Dogs constitute the main domestic link of visceral leishmaniasis, being considered the main source of infection for vectors. There is a need to correctly diagnose sick animals with and without clinical manifestation (asymptomatic). Methods and kits are provided to detect canine visceral leishmaniasis with high sensitivity and specificity, even in apparently healthy animals without clinical manifestations, using isolated *L. infantum* lipophosphoglycans. The developed method has a sensitivity of 91.7%, a specificity of 98.5%, an accuracy of 99.7%, with the ability to properly discriminate sera from infected dogs sick from those parasitized and clinically healthy, and with a low cross-reactivity index when they were Samples of dogs infected with other infectious agents were tested.

## Description

### Field of the invention

The invention relates generally to a method of diagnosing leishmaniasis and to a diagnostic kit. More specifically, the invention relates to the immunological detection of canine visceral leishmaniasis, with high sensitivity and specificity in the detection of infected animals, including the asymptomatic dogs of endemic area.

### Background of the Invention

Organisms of the Leishmania genus are macrophage parasitic protozoans with a high capacity to evade the host's immune system, causing different clinical forms of leishmaniasis in humans and domestic animals, primarily dogs.

Visceral leishmaniasis is a chronic anthropozoonosis. The etiological agent in India and Central Africa is *Leishmania donovani,* while in the Middle East, China, Mediterranean and the Americas it is *Leishmania infantum* (syn. *Leishmania chagasi*) (WHO. WORLD HEALTH ORGANIZATION. Control of the leishmaniasis: report of a WHO Expert Committee. World Health Organization, Geneva. Technical Report Series, 2010). Recently, the isolation of *Leishmania amazonensis* in dogs with clinical manifestations of visceral leishmaniasis in Governador Valadares, MG, Brazil has been described, indicating the involvement of the species with this disease (Valdivia HO et al. Comparative genomics of canine-isolated Leishmania (Leishmania) amazonensis from an endemic focus of visceral leishmaniasis in Governador Valadares, southeastern Brazil. Sci Rep. 7:40804, 2017).

*Leishmania* 's potential wild reservoirs are foxes and opossums, and the main domestic reservoir is the *dog(Canis familiaris*)*,* which has been identified as the main source of infection for the vector (BRAZIL, 2014). Ministry of Health. Secretariat of Health Surveillance. Department of Epidemiological Surveillance. Manual de vigilância e controle da leishmaniose visceral/Ministério da Saúde, Secretaria de Vigilância em Saúde, Departamento de Vigilância Epidemiológica. - 1. ed., 5. reimpr. - Brasilia: Ministry of Health). Outbreaks of human infections are commonly associated with the presence of infected dogs and vectors.

Canine visceral leishmaniasis (CVL) is a serious, slowly evolving systemic disease. The clinical picture of CVL presents a spectrum of characteristics that varies from the apparently healthy state (stage I) to a severe symptomatic state (stages II-IV). In the stage I, the seropositive dogs present themselves without clinical signs or with light clinical signs of the disease, as peripheral lymphadenomegaly or papular dermatitis, without alterations in the laboratorial parameters. In the stage II the dogs can present diffuse or symmetrical skin lesions as exfoliative dermatitis/onicogyphosis, ulcerations, anorexia, weight loss, fever and epistaxis. In the laboratory, the CBC shows anemia and the proteinogram with significant changes such as hypoalbuminemia and hypergammaglobulinemia, reduced values of the albumin: globulin ratio. However, the renal profile remains preserved. In addition to the changes mentioned above, there may be immunocomplex lesions such as vasculitis, arthritis, uveitis and glomerulonephritis. At this stage, values compatible with kidney damage already appear in the clinical laboratory. In stage IV, there is more serious organic involvement, pulmonary thromboembolism, or nephrotic syndrome and end-stage renal disease. In the laboratory, changes in the CBC are evident, such as severe anemia without regeneration. In serum and urinary biochemistry there is severe impairment of organs important for animal survival (Solano-Gallego L et al. Directions for the diagnosis, clinical staging, treatment and prevention of canine leishmaniosis - Review. Vet Parasitol. 165: 1-18, 2009).

The variation in clinical symptoms of infected dogs is related to hematological, biochemical changes, antibody titers, parasite load and vector insect infectivity (Costa-Val AP da, et al. Canine visceral leishmaniasis: relationships between clinical status, humoral immune response, haematology and Lutzomyia (Lutzomyia) longipalpis infectivity. Vet J. 2007;174(3):636-643 Manna L, et al. Evidence for a relationship between Leishmania load and clinical manifestations. Res Vet Sci. 2009;87(1); Freitas JC de, et al. Profile of anti-Leishmania antibodies related to clinical picture in canine visceral leishmaniasis. Res Vet Sci. 2012;93(2):705-709; Manzillo VF, et al.Prospective study on the incidence and progression of clinical signs in naive dogs naturally infected by Leishmania infantum. PLoS Negl Trop Dis. 2013;7(5):e2225; Ribeiro RR, et al. Relationship between clinical and pathological signs and severity of canine leishmaniasis. Rev Bras Parasitol Vet. 2013;22(3):373-378).

It is important to note that in areas endemic for CVL in Brazil the percentage of asymptomatic dogs can reach 70% (Abbehusen MMC, et al. Clinical and immunopathological findings during long term follow-up in Leishmania infantum experimentally infected dogs. Sci Rep. 2017 7(1):15914). These dogs, although asymptomatic, are reservoirs of the disease, being potentially able to infect the insect vector(Laurenti MD, et al. Asymptomatic dogs are highly competent to transmit Leishmania (Leishmania) infantum chagasito the natural vector. Vet Parasitol 2013 196: 296-300).

Accurate diagnosis of LVC is a fundamental tool for early treatment and control of the disease. This requires the development of tests with high accuracy, low rates of false negatives, capable of diagnosing dogs without clinical signs (asymptomatic). This is especially relevant in Brazil, due to the high percentage of asymptomatic dogs in endemic areas (Abbehusen MMC, et al. Clinical and immunopathological findings during long term follow-up in Leishmania infantum experimentally infected dogs. Sci Rep. 2017 7(1):15914). It is worth noting that according to data from IBGE the dog population in Brazil is 52 million dogs (National health survey: 2013: access and use of health services, accidents and violence: Brazil, large regions and federation units / IBGE , Coordination of Work and Income. - Rio de Janeiro : IBGE, 2015).

In practice, LVC diagnosis is performed via serological tests. Where possible, parasitological or molecular tests help to identify the *Leishmania* species responsible for the infection. When available, serological and parasitological data, preferably associated with clinical and epidemiological parameters, corroborate the diagnosis.

Parasitological tests consist of direct detection of the *Leishmania* parasite in organ aspirates, being a complex and invasive diagnosis. For this purpose, spleen, liver or bone marrow aspirations must be obtained, which requires professionals with medical or veterinary training. Such diagnosis is made by microscopic examinations on the samples collected and detection of amastigotes forms of the parasites (Reus M, et al. 2005. Visceral leishmaniasis: diagnosis by ultrasound-guided fine needle aspiration of an axillary node. Br J Radiol 78:158-160; Artan R, et al. 2006. Liver biopsy in the diagnosis of visceral leishmaniasis. J Gastroenterol Hepatol 21: 299-302). The specificity of the test is high, but the sensitivity is variable and depends on the presence of the parasite in the sample collected, as well as the technical capacity of the professional and the quality of the prepared slides (Del Olmo ML, et al. 2009. Visceral leishmaniasis diagnosed by duodenal biopsy. Rev Esp Enferm Dig 101: 439-440). Due to the low parasitemia, these tests have less sensitivity to asymptomatic animals. In a study of 1000 samples of lymph node and bone marrow aspirate, for example, only 28% of asymptomatic dogs were identified (Saridomichelakis MN, et al. Evaluation of lymph node and bone marrow cytology in the diagnosis of canine leishmaniasis(Leishrnania infantum) in symptomatic and asymptomatic dogs. Amer Jour of Trop Med and Hyg 2005; 73:82-6). Seeking an improvement in sensitivity, molecular methods can be associated with parasitological methods, increasing the chances of detecting the parasite DNA when it is in low amount in the tissues.

Although molecular diagnostic methods have high sensitivity for LVC diagnosis, their use is restricted to modern diagnostic laboratories, research centers and universities as they require highly trained professionals, infrastructure and specific machinery (Antinori S, et al. 2007. Clinical use of polymerase chain reaction performed on peripheral blood and bone marrow samples for the diagnosis and monitoring of visceral leishmaniasis in HIV-infected and HIV-uninfected patients: a single-center, 8-year experience in Italy and review of the literature. Clin Infect Dis 44:1602-10). Although the test itself presents high sensitivity and specificity, the sample to be used remains an important limitation, because there is a need for the presence of the parasite in the analyzed tissue. This may be in one or more tissues depending on the stage of infection and host immunity, e.g. skin, blood, spleen or marrow. Thus, the ease of detection of the parasite is variable. (BARROUIN-MELO, S.M.; LARANGEIRA, D.F.; TRIGO, J. et al. Comparison between splenic and lymph node aspirations as sampling methods for the parasitological detection of Leishmania chagasi infection in dogs. Memoirs of the Oswaldo Cruz Institute, v. 99, p. 95-197, 2004). Therefore, there are sensitivity problems directly linked to the sample used, as well as problems regarding the invasiveness of the sample collection process. Furthermore, although molecular tests have greater sensitivity for the diagnosis of asymptomatic dogs (Nunes CM, et al. Serological, parasitological and molecular tests for canine visceral leishmaniosis diagnosis in a longitudinal study. Rev Bras Parasitol Vet. 2015;24(4):402-9), the high cost and impossibility of making the diagnosis in field activities and veterinary clinics restrict the use of these tests in preventive actions in public health, especially in developing countries.

Since the 1990s, several serological tests have been developed for the diagnosis of visceral leishmaniasis, including LVC (Salotra P., Singh R. Rapid and reliable diagnostic tests for visceral leishmaniasis. Indian J Med Res. 2005; 122:464-7). Specific antibodies can be detected in direct agglutination tests (DAT), indirect immunofluorescence reaction (IFAT), immunoenzymatic assays (ELISA), western blotting, immunochromatography and, more recently, flow cytometry (Travi BL, Wild Lamb A, Dantas-Torres F, Myro on G (2018) Visceral canine leishmaniasis: Diagnosis and management of the reservoir living among us. PLoS Negl Trop Dis 12(1): e0006082).

Serological tests use complete or soluble extracts of the parasite, isolated specific molecules or recombinant proteins as antigens. The use of complete or soluble extracts limits considerably the specificity of immunological assays and makes standardization difficult, generating restrictions to these tests (ALVAR et al., 2004). Canine Leishmaniasis. Adv in Parasitol, v. 57, p. 88). Another limitation of raw antigens is the cross-reactivity of antibodies to other endemic diseases that occur in areas with LVC. Thus, recombinant proteins have been considered the most promising antigens for serodiagnostic assays (GOMES, Y.M.; PAIVA CAVALCANTI, M.; LIRA, R.A.; ABATH, F.G.C.; ALVES, L.C. Diagnosis of visceral canine leishmaniasis: Biotechnological advances. Review. The Veterinary Journal, v. 175, p. 45-52, 2008).

Among the main recombinant antigens used in the diagnosis of LVC we can mention the K39 protein (rK39). rK39 is a protein related to *L. infantum* kinesinase comprising repetitive sequences of 39 amino acid residues, used in the commercial kit recommended by the WHO, the Dipstick rK39®. However, the sensitivity and specificity of rK39 to detect cases of *L. infantum* infection varies according to the strain of the parasite, the production process of recombinant protein and the clinical and serological variability of patients in different countries (Zijlstra EE and El-Hassan AM. 2001. Leishmaniasis in Sudan: post kala-azar dermal leishmaniasis. Trans R Soe Trop Med Hyg. 95:S59-S76). In addition, rK39 presents low sensitivity for the diagnosis of asymptomatic infections in dogs (Santarem N, et al. Application of an Improved Enzyme-Linked Immunosorbent Assay Method for Serological Diagnosis of Canine Leishmaniasis. Jour of Clin Microbiol 2010 48:1866-74; Rhalem A, et al. Immune response against Leishmania antigens in dogs naturally and experimentally infected with L. infantum. Vet Parasitol 1999 81: 173-84).

Subsequently, other antigens were associated with rK39 in anattempt to obtain a test with greater specificity and sensitivity. DPP-LVC® is a rapid test based on the detection of k26/k39 antigens of amastigotes forms of the parasite. The test does not require the use of a laboratory structure and the Brazilian Ministry of Health recommends its use for screening animals with LVC (followed by the ELISA EIE-LVC test for confirmation of results). (BRAZIL, 2011. Ministry of Health, Health Surveillance Secretariat. Department of Communicable Disease Surveillance. Joint technical note no. 01/2011, p. 3). However, these tests have low sensitivity for the diagnosis of asymptomatic dogs (Grimaldi JG, et al. Evaluation of a novel chromatographic immunoassay based on Dual-Path Plat-form technology (DPP CVL rapid test) for the serodiagnosis of visceral canine leishmaniasis. Trans R Soc Trop Med Hyg 2012 106: 54-9; Lopes EG, et al. Serological and molecular diagnostic tests for canine visceral leishmaniasis in Brazilian endemic area: one out of five seronegative dogs are infected. Epidemiol Infect. 2017;145(12):2436-44;Pacheco LV, et al. Comparison of the tests recommended by the Ministry of Health for the diagnosis of canine visceral leishmaniasis. Rev de Educ Contin in Med Vet and Zootec of CRMV-SP, 2014 v. 12, n. 3; Belo VS, et al. Reliability of techniques used in the diagnosis of visceral canine leishmaniasis by the national control program in Brazil: A survey in an area of recent transmission. Preventive Vet Med. 2017 Vol 146, P 10-5).

The A2 protein, specific to the amastigota stage of *Leishmania,* is the antigen used in the LVC vaccine available in Brazil. Its use was evaluated for diagnostic use, however this antigen demonstrated inadequate sensitivity to identify asymptomatic animals (Carvalho AMRS, et al. An ELISA immunoassay employing a conserved Leishmania hypothetical protein for the serodiagnosis of visceral and tegumentary leishmaniasis in dogs and humans. Cellular Immunology. 2017 Vol 318, p 42-8).

The synthesis of chimeric proteins has been a strategy used to increase the sensitivity of the diagnosis of asymptomatic dogs (RA, Veloso LD, Coura-Vital W, Reis AB, Damasceno LM, et al. (2015) Novel Recombinant Multiepitope Proteins for the Diagnosis of Asymptomatic Leishmania infantum-Infected Dogs. PLoS Negl Trop Dis 9). The disadvantages of this technique are the costly and time-consuming process of synthesis, as well as the low stability of the synthesized compounds. Therefore, the development of more stable and sensitive antigens could help to solve these problems.

Serologic tests commonly used for diagnosis of LVC have been criticized for not being able to identify asymptomatic dogs. These tests generally do not have adequate sensitivity and specificity to detect the low levels of antibodies in these animals. Thus, a percentage of the samples are characterized as false negatives and/or inconclusive and additional tests, usually molecular tests, are required (Evaluating the accuracy of molecular diagnostic testing for visceral canine leishmaniasis using latent class analysis. PLoS One 9, e103635 (2014). In addition, these tests generally show cross reactions, not being able to differentiate infected dogs with other etiologies, such as *Trypanosoma cruzi, Ehrlichia canis, Toxoplasma gondii, Neospora caninum, Babesia canis* e *Leishmania braziliensis* (ZANETTE et al. 2014. Serological cross-reactivity of Trypanosoma cruzi, Ehrlichia canis, Toxoplasma gondii, Neospora caninum and Babesia canis to Leishmania infantum chagasi tests in dogs. Rev da Soc Bras de Med Trop, v. 47, n. 1:105-7;LIMA, V.M.F. DE; FATTORI, K.R.; MICHELIN, A.F.; SILVEIRA NETO, L.; VASCONCELOS, R.O. Comparison between ELISA using total antigen and immunochromatography with antigen rK39 in the diagnosis of canine visceral leishmaniasis. Veterinary Parasitology, v. 173, p. 330-333, 2010; SILVA et al. 2011. Laboratory tests performed on Leishmania seroreactive dogs euthanized by the leishmaniasis control program. Vet Parasitol, v. 179:257-61).

As the treatment for LVC-positive dogs was recently released in Brazil (MAPA, 2016, NOTA TÉCNICA N° 11/2016/CPV/DFIP/SDA/GM/MAPA.), a diagnosis that detects dogs in early stages of the disease would provide earlier treatment, increasing the chances of cure. This would also prevent the development of *Leishmania* resistance to the treatment drug and decrease the potential for transmission to the vectors. This treatment, besides being expensive and long, has high toxic potential, so the correct identification of positive animals is also important. This would prevent negative animals (identified as false-positive in diagnostic tests) from being referred for treatment unnecessarily.

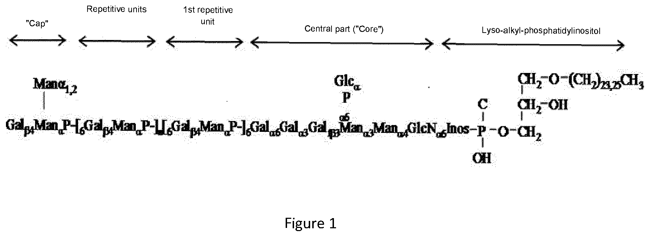

In this invention, lipophosphoglycans (LPGs) from *Leishmania* sp. are used, plus specifically from *L. infantum,* as diagnostic antigen for LVC. LPGs are the most studied and most abundant *Leishmania* surface glycol conjugate, forming a dense glycolocalyx that covers the entire surface of the parasite and scourge. The structure of the LPG has been described in several dermotropic and viscerotopic species of *Leishmania.* Basically, these molecules have a conserved central region of Gal(α1.6)Gal(α1,3)Galf(β1,3)[Glc(α1)-P04]Man(α1,3)Man(α1,4)-GlcN(αl), connected to a 1-*O*-alkyl-2-lysophosphatidylinositol anchor. The most striking part of the LPG is the domain of Gal's repetitive units (β1.4)Man(α1)-PO₄. This domain may have 16-30 repetitive units which give the molecule a considerable size. It is also in this portion that polymorphisms are found among the species of *Leishmania,* which are characterized by the variability of the composition of sugars linked to the repetitive units Gal(β1.4)Man(α1)-PO4 (Figure 1) (revised by Assis et al., 2012, Glycoconjugates in New World species of Leishmania: Polymorphisms in Lipophosphoglycan and Glycoinositolphospholipids and Interaction with Hosts Biochimica and Biophysics Acta General Subjects 1820: 1354-1365).

There are reports on the state of the art of using LPG as an antigen in diagnostic tests for human visceral leishmaniasis (Singh S. Changing trends in the epidemiology, clinical presentation, and diagnosis of Leishmania-HIVco-infection in India. Internat Journ of Infect Diseases Vol. 29, p. 103-12; Maalej IA, et al. Comparative evaluation of ELISAs based on ten recombinant or purified leishmania antigens for the serodiagnosis of Mediterranean visceral leishmaniasis. Am. J. Trop. Med. Hyg., 68(3), 2003, pp. 312-20) and the use of epitopes from the LPG cap region for LVC diagnosis (Anish C, et al. Immunogenicity and Diagnostic Potential of Synthetic Antigenic Cell Surface Glycans of Leishmania. ACS Chem. Biol., 2013, 8 (11), pp 2412-22).

However, in these works there is no evidence that LPG, unlike several antigens already tested in the state of the art, would be able to diagnose asymptomatic dogs with LVC. It is worth mentioning that Anish et al. demonstrated that it is not possible to diagnose LVC in asymptomatic dogs using *L. donovani* 's LPG *cap* (Anish C, et al. Immunogenicity and Diagnostic Potential of Synthetic Antigenic Cell Surface Glycans of Leishmania. ACS Chem. Biol., 2013, 8 (11), pp 2412-22).

As already described, despite the diversity of antigens and techniques available, there is still no ideal diagnostic method for LVC. In 2018, improving the ability to differentiate sick from clinically healthy/symptomatic dogs is still considered to be the remaining challenge for serological tests for LVC (Travi BL, et al. Canine visceral leishmaniasis: Diagnosis and management of the reservoir living among us PLoS Negl Trop Dis. 2018; 12(1).

The present invention solves this remaining problem of the state of the art, the detection of asymptomatic dogs. In this invention, lipophosphoglycans (LPGs) from *Leishmania* sp. are used, plus specifically LPG I from *L. infantum,* as diagnostic antigen for LVC. The invention uses the entire LPG molecule, extracted and purified from the parasite, as an antigen. Unlike Anish et al, who synthesized only the LPG Cap portion of *L. donovani* (Anish C, et al. Immunogenicity and Diagnostic Potential of Synthetic Antigenic Cell Surface Glycans of Leishmania. ACS Chem. Biol., 2013, 8 (11), pp 2412-22), The developed test was able to diagnose 90% of asymptomatic dogs of endemic area with high sensitivity.

Because it is based on carbohydrates, LPG has high stability, degrading only after 5 minutes at 100oC in the presence of 0.02 N hydrochloric acid (Rabbit-Finamore et al., 2011, Leishmania infantum: Lipophosphoglycan intraspecific variation and interaction with vertebrate and invertebrate hosts. International Journal for Parasitology 41: 333-342). This is an important advantage when compared to protein antigens, which denature easily. Such stability also allows the development of point-of-care tests, easy to apply and store, since the antigen can be taken to the field by health professionals without the need for refrigeration. In addition, the LPG's hydrophobic lipid anchor allows this antigen to bind on any solid support, from ELISA plates to immunochromatographic test nitrocellulose membranes. Other advantages of LPG are its low production cost, no recombinant technology or chemical synthesis is required, and its high specificity, so that there is no cross reaction with other canine pathogens such as *T. cruzi, Erlichia* and *Babesia.* In this sense, the method developed, in a surprising way, allows the diagnosis of infected dogs, but clinically healthy (classified as stage I), including dogs from endemic areas.

The invention will be presented in more detail below.

### Summary of the Invention

In one aspect, a method is provided to detect antibodies against *Leishmania* in an individual, comprising:
(i) contacting a biological sample of the said individual with a lipophosphoglycan; and
(ii) detecting the binding of the said lipophosphoglycan with the anti-Leishmania antibody in the biological sample, using a detection agent, thus detecting *Leishmania* infection in the said individual.

In one embodiment, the individual is a dog, more preferably, an asymptomatic dog.

In another embodiment, the biological sample is selected from the group consisting of blood, serum, plasma, saliva, milk, cerebrospinal fluid, tear, colostrum and urine.

In another form of embodiment, the lipophosphoglycan is bonded, conjugated or immobilized on or to a solid support. Preferably, the solid holder is a microtiter plate.

In one embodiment, a detection agent is used, in which the detection agent is an antibody marked with a selected marker of an enzyme, a radioisotope, a fluorescent group, a luminescent group, biotin, or dye particles.

In one form of embodiment, the method also includes a step to compare the levels of lipophosphoglycan-specific antibodies to the control level, where the level of lipophosphoglycan-specific antibody above the control is indicative of *Leishmania* infection.

In another respect, a kit is provided for the detection of *Leishmania* infection in an individual's biological sample, comprising a *Leishmania* lipophosphoglycan, a marked detection agent, a set of positive and negative controls, and instructions for use.

In another aspect, lipophosphoglycan is provided to manufacture a kit to detect *Leishmania* in a biological sample from an individual, or a method to detect antibodies against *Leishmania* in an individual.

### Brief Description of Figures

**Figure 1****.** Basic biochemical structure of the LPG molecule. Gal = galactose. Man=manose, Glucose, GlcN= N-acetyl glucosamine and Inos=inositol.

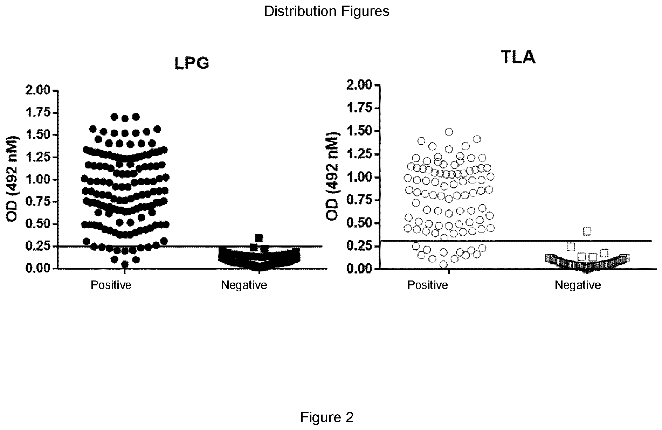

**Figure 2****.** Distribution of individual results for 165 positive and 97 negative serum samples. The sera were tested by indirect ELISA with total lysate antigen (ATL) from *Leishmania* and tested by indirect ELISA with lipophosphoglycan antigen (LPG) from *L. infantum* for identification of specific IgG antibodies in dogs: (A) ELISA-LPG and (B) ELISA-ATL. The line represents the*cut-off* point.

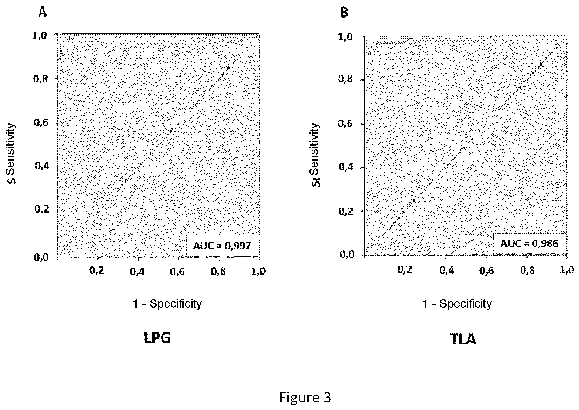

**Figure 3****.** ROC curve diagram of the ELISA test to determine the specificity of the tests for IgG anti *L. infantum.* Lipophosphoglycan or total lysed antigen was used as antigen. Analysis: SPSS v.12.0: (A) ELISA-LPG and (B) ELISA-ATL.

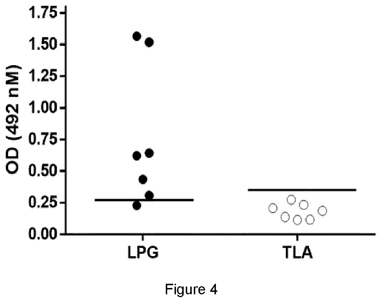

**Figure 4****.** Individual results for serum samples from clinically healthy and naturally infected dogs with *L. infantum.* The animals were previously identified as carriers of *L. infantum* infection by parasitological tests and tested by two different immunoenzymatic systems, for the identification of specific IgG antibodies to *L. infantum* in dogs: ELISA-LPG and ELISA-ALT. The line represents the cut-off point of the test.

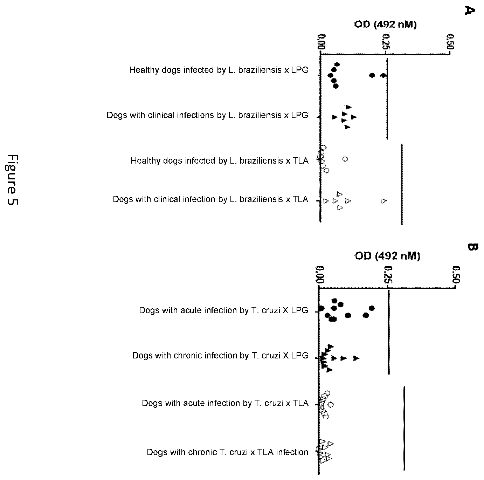

**Figure 5****.** Individual results for serum control samples from clinically healthy, parasitic and clinically diseased dogs naturally infected by *L. braziliensis* and experimentally by *T. cruzi.* The sera were tested with LPG and ATL (A) and (B) by indirect ELISA using LPG and TLA as antigens. The lines represent the cut-off point of the tests.

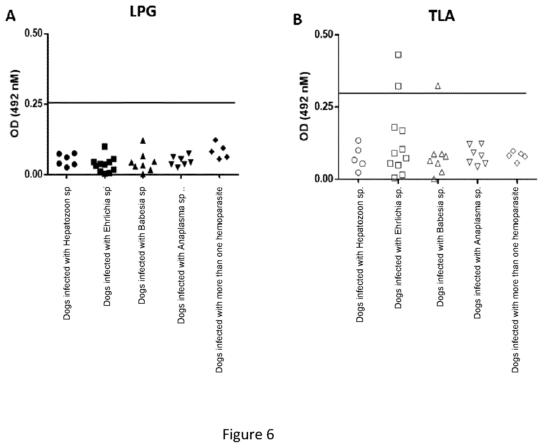

**Figure 6****.** Individual results for control sera samples from animals infected with *Hepatozoon* sp., *Anaplasma* sp., *Ehrlichia* sp., *Babesia* sp. and dogs presenting coinfection with 2 or 3 hemoparasites. For the identification of specific IgG antibodies to *L. infantum* in dogs: (A) ELISA-LPG and (B) ELISA-ATL. The line represents the cut-off point.

### Detailed Description of the Invention

Unless they are defined differently, all the technical and scientific terms used here have the same meaning understood by a technician on the subject to which the invention belongs. Conventional molecular biology and immunology techniques are well known to a technician on the subject. The descriptive report also provides definitions of terms to assist in interpreting what is described here and the claims. Unless otherwise stated, all figures expressing quantities, percentages and proportions, and other numerical values used in the descriptive report and the claims, should be understood to be modified, in all cases, by the term "about". Thus, unless otherwise stated, the numerical parameters shown in the descriptive report and the claims are approximations that may vary depending on the properties to be obtained.

The inventors of this work unexpectedly show, for the first time, the use of glycoloconjugate surface of *L. infantum,* the lipophosphoglycan (LPG), as an antigen in the immunodiagnosis of LVC capable of diagnosing clinically healthy (asymptomatic) dogs from endemic areas infected with the parasite.

Thus, the invention provides useful methods and kits to perform immune antigen detection for the diagnosis of canine visceral leishmaniasis.

As used here, "asymptomatic dog" refers to a dog that has been infected but is clinically healthy.

The term "antigen" must be understood in its broadest sense and can be any molecule, cell or particle. For example, an antigen includes, but is not limited to, a cell, polysaccharide, protein, nucleic acid and lipid, or a mixture of two or more of these.

An antigen can be in its pure form, or be in a mixture. An antigen may be in a modified form (for example, modified by chemicals), or it may be in an unmodified form.

"Antibody" should be understood in its broad meaning. An antibody is an immunoglobulin molecule that has the ability to bind specifically to a particular antigen. The antibodies are well known to a technician on the subject. An antibody includes, but is not limited to, a traditional antibody, a fragment of a traditional antibody containing an antigen binding site, a recombinant antibody containing an antigen binding site, a protein that binds to an antigen, and a product comprising a binding between two or more of these.

An antibody can be in its pure form, or be in a mixture. An antibody can be in a modified form (for example, modified by chemicals), or it can be in an unmodified form.

The term "detection agent" refers to an agent used to detect an antigen or antibody. A detection agent can be either an antigen or an antibody. A detection agent can be either a marked antigen or antibody or an unmarked antigen or antibody. Appropriate marking methods may be used in this invention and include, without limitation, isotope marking, chemical modification, enzyme conjugation, fluorescent dye marking, luminescent marking and other marking methods commonly known to the technician in the field. Therefore, a detection agent includes, but is not limited to, a chemical molecule, a peptide molecule, a protein molecule, an RNA molecule, a DNA molecule, a traditional antibody, a fragment of a traditional antibody containing an antigen binding site, a recombinant antibody containing an antigen binding site, a protein that binds to an antigen, a cell, a particle, and a product comprising a bond between two or more of the above.

A detection agent may be in pure form, or it may be in an impure form (for example, contained in a mixture with other compounds or materials). A detection agent can be in a modified or unmodified form. According to the order of a detection agent used in a method, a detection agent can be called 'primary detection agent', 'secondary detection agent', 'tertiary detection agent' and so on.

The term "detection system" refers to a system that can be used to provide a reading comprising information related to the quantity or quality of a protein or agent in a sample. The choice of a detection system depends on the choice of the detection agent used in a method of invention. For example, a detection system includes, but is not limited to, X-ray film, or other material sensitive to beta/gamma radiation if the detection agent is marked with an isotope; a CCD camera or visual inspection or other device capable of perceiving the signal if the detection agent is marked with an enzyme and generates a chemical reaction that may result in a colored signal or chemiluminescent signal capable of being detected; and if the detection agent is marked with fluorescence, a microscope, scanner or fluorescence camera may be used.

"Support" or "solid phase", used interchangeably, refers to a phase that connects to a target to be detected. Examples of such media include, but are not limited to membranes such as nitrocellulose, PVDF or nylon, a solid phase for ELISA such as polyacrylamide, plastic material such as polystyrene or polyvinylchloride, metal, glass or similar, and tissue samples such as tissue or cells in glass, plastic or other materials. Alternatively, the stand can also be a microsphere, magnetic particle or an optical sensor fiber as described in patent no. US 5,359,681.

A 'blocking agent' refers to a reagent that binds to non-specific binding sites on a substrate. Examples include Tween-20, BSA or combinations thereof. In one respect, BSA may be replaced by, for example, casein or other agents known in the art.

The invention provides methods and kits for the detection of an antibody against *Leishmania,* and the use of the antigen for the diagnosis of canine visceral leishmaniasis.

Thus, in one aspect, in the methods of the present invention, an antigen is immobilized to a solid support and contacted with a first detection agent, thus forming a complex antigen first detection agent. In a form of embodiment, the first detection agent is an antibody present in the test sample. The solid holder is then contacted with a solution comprising a second detection agent (a second antibody or anti-first antibody), attached to a marker. The first and second detection agents form a complex, and the antibody is qualitatively and/or quantitatively detected. In one application, the method is useful for the detection of antigen-specific antibodies using any immunological detection methods, including, but not limited to, Western blots, Dot blots, ELISA, lateral flow immunochromatography, immunofluorescence, flow cytometry. See, for example, Harlow and Lane. 1988. Antibodies, A Laboratory Manual, Cold Spring Harbor Publications, New York, for a description of immunoassay formats. In a preferred embodiment, an antibody detection method is provided by ELISA, and even more preferably, by indirect ELISA.

Indirect ELISA is an ELISA method that can be used for screening and determination of antibody titers during the course of their production. To detect a detection agent (e.g. an antibody) that recognizes an antigen, the antigen is coated on the substrate or solid phase, e.g. in wells of the microtiter plates, and incubated with test solutions containing specific detection agents. Unbound detection agents are washed away. A second incubation with a solution containing a secondary detection agent (e.g. alkaline phosphatase conjugated to protein A, protein G or antibodies which bind to the primary detection agent) is then required. After incubation, the unbound secondary detection agent is washed away. An incubation with a reporter enzyme substrate may also be necessary. A detection system such as UV, fluorescence, chemiluminescence or other methods is used to detect the binding secondary detection agent, which is proportional to the amount of detection agent to be detected in the test solution.

The method of this invention comprises:
- Contac a biological sample with the antigen of this invention, and
- Detect the binding of the said antigen with antibody present in the said biological sample.

Optionally, before the step of contacting the biological sample with the antigen, the method may also comprise a sensitization step or immobilization of the antigen in the solid support where the method occurs, and a blocking step of this same solid support. Also, before the detection step of the binding agent in the biological sample, the method may comprise a binding step of a second detection agent.

In other words, in the optional form, the method comprises:
- Sensitize a solid phase with an antigen,
- Block the solid phase with a blocking reagent,
- Contact a biological sample comprising a first detection agent with the solid phase of the previous step,
- Incubate the solid phase of the previous step with a second detection agent, and
- Detect the connection of the second detection agent in the solid phase with a detection system.

In an embodiment of the invention, the antigen can be a protozoan cell, whole or lysed. Specifically, the antigen is the protozoan of the genus *Leishmania,* more specifically, Leishmania *infantum,* a glyconjugate of Leishmania *infantum,* and more specifically, a lipophosphoglycan of Leishmania *infantum.*

For the purpose of this invention, "lipophosphoglycans" (LPGs) are molecules present in high amounts in the membrane of *Leishmania,* which form a dense glycocalyx that covers the entire surface of the parasite and scourge (ASSIS et al. 2012. Glycoconjugates in New World species of Leishmania: Polymorphisms in lipophosphoglycan and glycoinositolphospholipids and interaction with hosts, Biochimica et Biophysica Acta, v. 1820 p. 1354-1365; FRANCO et al. 2012. Innate Immune Activation and Subversion of Mammalian Functions by Leishmania Lipophosphoglycan Review Article. Journal of Parasitology Research, v. 2012, Article ID 165126, 11 pages).

LPGs can be extracted and purified from a *Leishmania* cell culture. To obtain the culture, biological material can be inoculated, for example, but not limited to splenic aspiration, from spleen, bone marrow and lymph nodes, from individuals infected with *Leishmania* in medium suitable for parasite multiplication, such as, but not limited to, biphasic medium comprising solid NNN Novy-MacNeil-Nicole (NNN) medium (rabbit blood agar) and liquid Schneider's medium (Sigma Chemical Co., St. Louis). Louis, USA) supplemented with bovine fetal serum, and maintained at 20 to 25°C for 20 to 40 days. Another example of an *in vitro Leishmania* cultivation process is described, for example, in patent No. US6458581. The technician in the field can easily define other means and conditions more suitable for growing the parasites. Once the culture is obtained, preferably from promastigotes forms of parasites, LPGs are extracted by methods such as those described by Orlandi & Turco (ORLANDI, P.A.; TURCO, S.J. 1999. Structure of the lipid moiety of the Leishmania donovani lipophosphoglycan. Journal of Biological Chemistry, v. 262, p. 10384 - 10391). Other methods of knowledge of the technician on the subject can be used.

The antigens of this invention can also be obtained synthetically, for example, as described by methods described in art (e.g. AL-MAHARIK N. et al. 2010. Chemical synthesis of parasitic glycoconjugates and phosphoglycans. Chapter 26. In: Microbial Glycobiology, Structure, Relevance and Applications. p. 477-548; HIGSON A. 2005. Synthetic fragments of antigenic lipophosphoglycans from Leishmania major and Leishmania mexicana and their use for characterization of the Leishmania elongating α-D-mannopyranosylphosphate transferase. Chemistry, v. 11, n. 7, p. 2019-30).

The solid support can be sensitized with the antigens using a variety of techniques known to the technician on the subject, which are widely described in art. In the context of this invention, the term "sensitization", used interchangeably with the term "immobilization", refers to both the non-covalent association, such as adsorption, and the covalent bond (which can be via a direct link between the antigen and functional groups on the support, or can be a link by means of a cross-linking agent). For this purpose, the antigen is dissolved in a suitable buffer, here called "solubilization buffer", which has an alkaline pH typically ranging from 7.0 to 10.0, but preferably about 9.0 to 10.0. An example is the carbonate/bicarbonate buffer, but the person skilled in the art can easily determine other buffers, according to the different protocols known in the art. The contact time of the antigen with the solid support varies with temperature, but is typically between about 1 hour to overnight, in temperature ranging from about 2°C to about 5°C.

In another embodiment, in the blocking step of the solid phase, blocking agents are used The blocking step blocks any hydrophobic binding sites in the solid phase to avoid non-specific binding of proteins, thus reducing the background signal and/or preventing false-positive results.

Examples of blocking agents include, but are not limited to skim milk, casein, BSA, ovoalbumin, gelatine, among others. The blocking agent is diluted in a buffer, e.g. PBS buffer, generating the blocking solution. Other plugs of the technique can be used. The pH of the blocking solution can be in the range of 7.0 to 10.0, but preferably around 7.0 to 9.0. The contact time of this solution with the solid support varies from about 30 minutes to about 6 hours, at temperature ranging from about 25° C to about 40° C, more preferably, about 30 ° C to about 40° C.

In an additional embodiment, once the solid phase is blocked, it is put in contact with a detection agent. In this sense, the method of this invention detects dogs with visceral leishmaniasis through the use of a biological sample from a dog that has been potentially exposed to *Leishmania,* more specifically, *L infantum.* The biological sample can be any sample taken from the body which may contain a detection agent. Such biological samples can be selected from the group including blood, serum, plasma, saliva, milk, colostrum and urine. Preferably, the biological sample is blood, serum and plasma.

It is also preferable that the biological sample be obtained from individuals suspected of exposure to *Leishmania.* A biological sample can also be modified before use, such as by dilution, purification into various fractions, centrifugation and the like. For example, the biological sample containing the first detection agent can be used in pure form or diluted in a buffer, such as the blocking buffer from the previous step.

It should be noted that a biological sample may be free of a detection agent that may interact with *Leishmania.* This occurs when the subject has not been exposed to *Leishmania.*

In an embodiment, the biological sample is applied to a solid support sensitised with LPG of *Leishmania infantum.* The biological sample shall be left for a sufficient period of time and under appropriate conditions allowing stable formation of a primary detection antigen complex. Typically, the biological sample comprising the detection agent remains in contact with the solid support for about 30 minutes to about 6 hours, at a temperature ranging from about 25 ° C to about 40 ° C, more preferably, about 30°C at about 40° C.

Once the complex is formed, a secondary detection agent is added to detect the complex formed between the antigen and the primary detection system present in the biological sample.

The secondary detection agent is diluted in a buffer, such as the blocking buffer used previously. Typically, the secondary detection agent remains in contact with the solid support for about 30 minutes to about 6 hours, at a temperature ranging from about 25° C to 40° C, more preferably, about 30°C to about 40° C.

The primary and secondary detection agents are incubated in the solid phase separately.

Among the aforementioned steps, the solid support is washed with buffer at neutral pH, typically ranging from 7.0 to 7.4, for example, PBS, to avoid unspecific binding and thus reduce the background signal.

The person skilled in the art will know how to select a secondary detection agent for the antigen-primary detection agent complex. A secondary detection agent may be, but is not limited to one chemical molecule, one peptide molecule, one protein molecule, one RNA molecule, one DNA molecule, one poly or monoclonal antibody, one antibody fragment containing an antigen binding site, one recombinant antibody containing an antigen binding site.

A commonly used detection agent comprises polypeptides A, G, L, A/G which bind to antibodies. These polypeptides can bind to the preserved antibody region. Other commonly used detection agents are avidin or streptavidin, which can bind to biotinilated antibodies or polypeptide. The detection agent can be marked or unmarked.

Another most commonly used detection agent is an antibody. Antibodies can be derived from different species, and include, but are not limited to, dogs, humans, cats, opossums, foxes, rats, mice, birds, among others. Antibodies commercially available to a wide variety of antigens are known in the art. Thus, for example, if the method is used to diagnose dogs, commercial dog antibodies, e.g. anti-clog IgG, are used.

The person skilled in the art knows that there are a variety of marking methods for a detection agent.. Marking methods include, but are not limited to, an enzyme such as horseradish peroxidase (HRP), alkaline phosphatase (AP), beta-galactosidase or other enzymes. A detection agent can also be marked with radioactive iodine isotopes or other isotopes. A detection agent can also be marked with a fluorochrome (a fluorescent dye) that can be detected by a fluorescence microscope or fluorometer, scanner or camera. Alternatively, a detection agent can be marked by biotin, which can be linked to avidin or streptavidin.

The detection of the antigen complex :: primary detection agent:: secondary detection agent can be based on a method known in the art. The person skilled in the art knows the different detection systems used in ELISA and which can be applied to the methods of the invention described herein. These detection systems include, but are not limited to, detection systems using chromogenic reactions or reporter enzymes such as horseradish peroxidase (HRP) and alkaline phosphatase (AP). Reporter enzymes can use different substrates for chromogenic detection. For example, HRP can use OPD (orthophenylenediamine), 4CN (4-chloro-1-naphthol), DAB/NiCl2 (3,3'-diaminobenzidine/NiCl2), or TMB as substrates for chromogenic detection.

A negative result is defined as the absence of a signal emitted by the marked detection agent, comparable to a negative control (e.g. biological material coming from an individual who has not been exposed to *Leishmania*). In contrast, a signal level emitted by the marked detection agent above the control is indicative of *Leishmania* infection.

In the methods of the present invention, the person skilled in the art knows of the modifications to be made to improve the signal / background signal ratio. These modifications include, but are not limited to, adding one or multiple steps to the method described above.

In another aspect, a kit comprising one or more useful components for performing an ELISA and instructions for using the kit is provided. For example, such instructions may include one or more components to perform the method. For example, the kit may include negative and/or positive control, marked secondary detection agent, and any other reagents needed to conduct the diagnostic method described here.

The present invention is described by the non-limiting examples below, which are merely illustrative. Various modifications and variations of the embodiments are evident to the person skilled in the art, without departing from the spirit and scope of the invention.

### EXAMPLES

### Example 1: Samples used

For standardization of the indirect ELISA, samples from 97 dogs were used and had as inclusion criteria: positive parasitological tests for amastigotes in lymph nodes and bone marrow, promastigotes in culture of splenic aspiration and positive detection of DNA of *L. infantum* by the PCR technique of splenic aspiration. Among the samples collected, there were groups of clinically healthy dogs that were even severely parasitic, representing the reality of LVC in Brazil. The 68 dogs included in this study and confirmed as negative were evaluated clinically and laboratorialy by the same tests as the positive animals and presented negative results, and came from non-endemic areas for LVC.

The control sera for cross reactivity testing on ELISA followed the inclusion criteria of direct visualization of parasites on blood smears or PCR of blood samples from dogs. The sera samples from dogs with *Trypanosoma cruzi* came from dogs experimentally infected with the parasite and were collected in the acute and chronic stages of the disease. Table 1 shows a small summary of the characteristics of serum samples used in this study.

**Table 1. Number of serum samples to perform LPG and ATL antigen cross-reaction ELISA assays.**

| Classification of groups of dogs with serum tested for cross reaction of IgG anti-Leishmania and other pathogens | n |
|---|---|
| Clinically healthy dogs, parasited by *Leishmania braziliensis* | 7 |
| Clinically diseased dogs, parasited by *Leishmania braziliensis* | 6 |
| Dogs experimentally infected with *Trypanosoma cruzi* (acute phase) | 20 |
| Dogs experimentally infected with *Trypanosoma cruzi* (chronic phase) | 20 |
| Dogs infected with *Hepatozoon* sp. | 6 |
| Dogs infected with *Ehrlichia* sp. | 4 |
| Dogs infected with *Babesia* sp. | 3 |
| Dogs infected with *Anaplasma* sp. | 2 |
| Dogs clinically coinfected by 2 or 3 haemoparasites | 5 |

### Example 2: Direct parasitological identification by cytology

The parasitological examinations were performed using samples obtained by the fine needle puncture technique (PAAF) of splenic aspirate (BARROUIN-MELO et al. 2006. Can spleen aspirations be safely used for the parasitological diagnosis of visceral canine leishmaniosis? A study on asymptomatic and polysymptomatic animals. The Veterinary Journal, v.171, p. 331-339), of bone marrow and lymph nodes. The animals were sedated with intravenous administration of 0.1 mg/Kg of acepromazine. The samples obtained from bone marrow and lymph nodes by puncture were extended on slides using the *squash* technique, air dried and submitted to staining with the Panotic LB® rapid kit (Laborclin, Paraná, Brazil). The samples were then observed under an optical microscope (Zeiss®, Germany) at a magnification of 1000x.

### Example 3: Cultivation of L. infantum from splenic aspirates

For the culture of *Leishmania* sp., from the splenic aspiration, approximately 100-200µL of each sample was inoculated in biphasic medium, containing 1.5mL of solid medium NNN Novy-MacNeil- Nicolle (NNN) (rabbit blood agar) and 2mL of liquid medium Schneider's (Sigma Chemical Co., St. Louis). Louis, USA) supplemented with 20% inactivated bovine fetal serum at 56°C for 30 minutes (Gibco, N.Y, USA) and gentamicin 50µg/mL. The crops were kept in a BOD oven at 23°C and examined weekly under light microscopy at 40X magnification for up to 30 days. The positive diagnosis was admitted by the visualization of promastigotic forms presenting active movement and the negative by the absence of promastigotic forms after 4 weeks of observation of the crops (Comparison between splenic and lymph node aspirations as sampling methods for the parasitological detection of Leishmania chagasi infection in dogs. Barrouin-Melo SM, Larangeira DF, J Wheat, Aguiar PH, dos-Santos WL, Oak Bridges L. Mem Inst Oswaldo Cruz. 2004 Mar;99(2): 195-7).

### Example 4: Extraction of DNA from the splenic aspirate and performance of the polymerase chain reaction (PCR) technique

The commercial Wizard Genomic DNA Purification *Kit*® (Invitrogen Life, Brazil) was used for DNA extraction and polymerase chain reaction (PCR) of splenic aspiration samples, following the manufacturer's instructions. The research of *L. infantum* DNA using the Polymerase Chain Reaction (PCR) technique was performed as described by LACHAUD et al. (2002) (LACHAUD, L.; MARCHERGUI-HAMMAMI, S.; CHABBERT, E. et al. Comparison of six PCR methods using peripheral blood for detection of visceral canine leishmaniasis. Journal of Clinical Microbiology, v. 40, p. 210-215, 2002.), using the primers RV1 (forward; 5'-CTTTCTGGTCCCGGTAG-3') and RV2 (reverse; 5'-CCACCTGGCCTATTTACCA-3') (Invitrogen Life, Brazil), specific for *L. infantum.*

### Example 5: L. infantum culture to obtain promastigotes L. infantum lysate antigen (ATL)

Promastigotes of Leishmania *(Leishmania) infantum* isolated from macerated spleen of hamsters chronically infected with the reference strain of *L. infantum,* were inoculated in biphasic medium NNN-Schneider's (Sigma-Aldrich Inc., St. Louis, USA). Louis, USA) supplemented with 20% heat inactivated bovine fetal serum at 56°C and added 50µg/mL of gentamicin. The parasites grew in 25cm2 culture bottles in triplicate and counted daily in Neubauer chamber. After 2 or 3 passages in culture of promastigotes in stationary phase, the cultures were washed by centrifugation 3 times with sterile PBS at 3000 rpm for 10 minutes and stored in a freezer at -80°C. For the preparation of the antigen, the culture sediments containing promastigote forms of the parasite frozen in liquid nitrogen were thawed and frozen at room temperature for 3 consecutive times, then sonicated at a power of 4 Hz in a 1 minute cycle (Sonic Desmembrator, Fischer Scientific , USA). Centrifugation at 14000rpm followed for 10 minutes at 4°C, the supernatant containing the soluble fraction of the extract was collected from which an aliquot was obtained for protein quantification by the Bradford method (Bio-Rad). This was aliquoted, cryopreserved at -20oC and used in indirect ELISA assays.

### Example 6: Purification of Lipophosphoglycan (LPG) from L. infantum

The Orlandi & Turco (1987) method was used to extract the glyconjugate. The parasites in promastigota form were washed in phosphate buffer (PBS) and centrifuged at 700 rpm at room temperature. To the sediment 2.5mL CHCl3/MeOH (3:2) and 0.5mL MgCl2 at 4mM was added which was later sonicated and centrifuged. To the intermediate solid phase 2.5mL of 4mM MgCl2 solution was added again, which was later sonicated and centrifuged. Then, 3.0mL CHCl3/MeOH/H20 (10:10:3) and 0.5mL CHCl3/MeOH (1:1) were added to the sediment, only for the first centrifugation. For LPG extraction 2.5mL of ESOAK (water/ethanol/ethyl ether/pyridine/NH₄OH; 15:15:5:1:0.017) followed by sonication and centrifugation were added. The supernatant containing LPG was evaporated, using nitrogen at 45°C. After this, the sample was resuspended in 1 mL of CH3COOH 0.1N / NaCl 0.1N, sonic and applied on a phenyl sepharose column.

Using a Bio-Rad column (#731-1550) about 2mL of phenyl-pharose were added, so that the final volume after packaging was between 0.6-1.0mL. The column was washed with approximately 6 volumes of CH₃COOH 0.1N / NaCl 0.1N to pack the resin. After the last mL of CH₃COOH 0.1N / NaCl 0.1N penetrated the column the sample containing the LPG was added. The material was then washed in the following sequence: 1mL CH3COOH 0.1N; 1mL ddH20 and 4mL ESOAK used to elute the already purified material. The fraction containing LPG was again evaporated in nitrogen in a bath at 45°C, resuspending in 1mL of milli-Q water. To confirm the purification of LPG, the ELISA technique was performed with the monoclonal antibody CA7AE.

### Example 7: Development of ELISA with total lysate antigen (ATL) and lipophosphoglycan (LPG)

The ELISA assay was developed based on the checkerboard procedure, first considering a fixed dilution of dog anti-IgG antibody conjugated to peroxidase, and varying the concentration of antigens (protein / glycoconjugate) and the dilution of the samples. In the second checkerboard, after defining the antigen concentrations and dilutions of the samples that gave the highest positive pool / negative pool ratio, different dilutions of the conjugate were tested.

The positive *pool* consisted of an equal number of ten positive control samples (as described previously in Table 1), and the negative *pool* was also a mixture of ten negative control samples.

The test was developed on 96-well polystyrene microplates (Parker Elmer, Waltham USA), which were sensitized with antigens diluted in carbonate/bicarbonate buffer pH 9.6 to 100µL/well and incubated at 4°C for 14 hours. The plates were washed four times with 0.05% Tween 20 (PBST) added PBS, blocked with 10% casein added PBST and incubated at 37°C for two hours. After four washes with PBST, 100µL of *pools* of sera diluted in 5% to 100uL PBST casein per well were added and incubated at 37°C for one hour. Each serum sample was tested in duplicate. The plates were washed with PBST four times and peroxidase conjugated dog IgG (Bethil, USA) diluted in 5% PBST casein was added to the plate, 100uL per well and incubated at 37°C for one hour. After incubation, the plates were washed four times with PBST and applied to each well 100µL of solution prepared with citrate buffer pH 5.3 plus 12µL H₂O₂ and 5mg orthophenylenediamine (OPD) (Sigma Aldrich) per plate. The reaction was stopped with 50µL per well of H₂SO₄ 4N.

The best concentrations of antigens, sample dilutions and peroxidase-conjugated dog IgG antibody for ELISA using LPG were 0.50µg/mL, 1:400 and 1:10,000, and for ATL were 0.50µg/mL, 1:500 and 1:20,000 respectively. The antigens, serum samples and dilution of IgG antidog conjugated with peroxidase chosen had an optical density ratio of positive/negative control sera of 17.99 on the LPG ELISA and on the ATL ELISA was 11.78. The distribution of optical densities (OD) is demonstrated in Figure 2, which identifies each sample of positive and negative control sera, and the horizontal line determines the cut-off point for each test.

### Example 8: Mathematical and statistical analysis

The cutoff point value for each ELISA was defined by the mean of the optical density (OD) of the negative control sera plus three standard deviations (FREY et al., 1988). Based on sera results in the ELISA, samples were classified into four different states: true positive (positive for parasite detection or PCR, and positive for the ELISA); true negative (negative for parasite detection or PCR, and negative for the ELISA); false positive (negative for the reference test and positive for the ELISA); and false negative (positive for the reference test and negative for the ELISA). With these results, the sensitivity (Se), specificity (Sp), positive predictive value (PPV), negative predictive value (NPV) and accuracy (Ac) parameters of the ELISA were determined for the detection of antibodies against total lysed antigen (ALT) and lipophosphoglycan (LPG) of *L. infantum* using the following formulas, where TP is truly positive, TN is truly negative, FP is false positive and FN is false negative: Sensitivity = TP / TP + FN; Specificity = TN / TN + FP; Predictive Value Positive = TP / TP + FP; Predictive Value Negative = TN / TN + FN; Accuracy = TP + TN / TP + FP + FN, (SEYFFERT et al., 2010).

The ROC*(Receiver Operating Characteristics)* curve was obtained using the SPSS v. 12.0 software that defined the accuracy from the area under the curve (AUC) formed. The level of agreement was classified using the Kappa (K) test as described by Landis and Koch (1977): 0 - no agreement; 0 to 0.19 - very low correlation, 0.20 to 0.39 - weak agreement, 0.40 to 0.59 - moderate agreement, 0.60 to 0.79 - substantial agreement and 0.80-1.00 - high agreement.

To check repeatability and reproducibility, a pool of positive and negative samples tested on three different days by different technicians of the immunology and cell biology laboratory (reproducibility) and twenty times on the same day (repeatability) under the same conditions was used. The results for these parameters are expressed as percentage (%) of agreement for both reproducibility and repeatability.

Table 2 shows the validation criteria for the ELISA with the number of sera samples used in the standardization. The cut-off point was defined from the mean of the negative controls plus three times the standard deviation, being 0.251 in the ELISA-LPG and 0.280 in the ELISA-ATL. It was possible to determine in the ELISA-LPG the presence of a false-positive result as well as in the ELISA-ATL. In the test that used the LPG as antigen a sensitivity of 91.7% was achieved while in the test with ATL it was 87.6%. The specificity of 98.5% and PPV 0.988 were established in both tests, however the VPN was 0.893 with LPG and 0.848 with ATL. The accuracy of the tests was established from the area under the ROC curve observed in Figure 3, where the data of the positive and negative controls in the production of the ROC curve were compiled, and for the test that used LPG it was 0.997, and 0.986 in the ATL.

The method that used the LPG antigen was quite stable, since the repetitivity was 97.5% and the average OD was 1.176 in the positive controls and when the results are compared to the method with ATL, the repetitivity was 97.1% with an average OD of 1.390 in the positive controls. The repetitivity of the negative controls was 89.7% and OD with an average of 0.038 for LPG, and for ATL was 85.4% and OD with an average of 0.068. The reproducibility of the LPG antigen test was 99.7% in the positive *pool* and 99.2% in the negative *pool* , values presented in Table 2. The level of agreement with Kappa test (K) was 0.9, classified as high according to Landis and Koch (1977).

Table 2. Validation of parameters found for ELISA tests developed for identification of specific IgG antibodies against *L. infantum* LPG and TLA antigens using parasite identification/isolation techniques or PCR for *L. infantum* as gold standard

| Parameters | LPG | TLA |
|---|---|---|
| No. of sample tested | 165 | 165 |
| Positive controls | 97 | 97 |
| Negative controls | 68 | 68 |
| Truly positive | 89 | 85 |
| Truly negative | 67 | 67 |
| False negatives | 8 | 12 |
| False positive | 1 | 1 |
| Cut off | 0.251 | 0.280 |
| Sensitivity (%) | 91.7 | 87.6 |
| Specificity (%) | 98.5 | 98.5 |
| Accuracy (AUC) | 0.997 | 0.986 |
| PPV (%) | 98.8 | 98.8 |
| VPN (%) | 89.3 | 84.8 |
| Kappa *(K*) | 0.9 | |
| Repeatability + (result) (%) | 97.5 | 97.1 |
| Repeatability + (DO) (average) | 1.176 | 1.390 |
| Repetitivity - (result) (%) | 89.7 | 86.4 |
| Repetitivity - (DO) (average) | 0.038 | 0.068 |

During clinical and laboratory evaluation of 97 positive dogs of endemic area, 7 clinically healthy and parasitic dogs were identified that presented clinical staging of mild disease, according to the classification of Solano-Gallego et al. (SOLANO-GALLEGO, L. et al. 2009. Directions for the diagnosis, clinical staging, treatment and prevention of canine leishmaniosis - Review. Veterinary Parasitology, v. 165, p. 1-18), they were submitted to ELISA test with ATL and it was observed that all the 7/7 (100%) dogs had DO below the *cut-off,* therefore considered negative. When tested against LPG, 6/7 (85.7%) of the dogs presented high DOs, being considered positive because they remained above the *cut-off* (Figure 4).

To verify cross-reactivity with other diseases that occur in LVC endemic regions, positive animal control sera were tested for the pathogens *Leishmania braziliensis, Trypanosoma cruzi, Hepatozoon* sp., *Ehrlichia* sp., *Babesia* sp. and *Anaplasma* spp.

Figure 5A shows the results of cross-reactions in the ELISA assays with LPG and ATL antigens tested with control sera samples from clinically healthy and parasitic dogs as well as clinically sick and infected *L*. *braziliensis.* In figure 4B, cross reaction test data using sera samples from *T. cruzi* artificially infected dogs in the acute and chronic phases of the disease were portrayed. In both evaluations, in the results obtained against the two antigens, no cross-reactivity against them was evidenced.

In Figure 6, possible cross-reactions with infectious agents were investigated, which may generate false positive results in the identification of *L. infantum* infected dogs. As it is represented in figure 5B, 18.2% (2/11) dogs with erliquiose and 12.5% (1/8%) dogs with babesiose presented cross reaction against ATL, but were considered negative when evaluated in ELISA-LPG.

### Example 9: Comparison with commercial art diagnostic tests

This method showed sensitivities (Se) of 91.7% in ELISA-LPG and 87.5% in ELISA-ATL, specificity (Sp) of 98.5% in both tests and accuracy of 0.997 and 0.986 against LPG and ATL, respectively. Compared to the results of De Arruda et al. (2013) (De ARRUDA, M.M.; FIGUEIREDO, F.B.; MARCELINO, A.P.; BARBOSA, J.R.; WERNECK, G.L.; NORONHA, E.F.; ROMERO, G.A.S. Sensitivity and specificity of parallel or serial serological testing for detection of canine Leishmania infection. Memoirs of Instituto Oswaldo Cruz, Rio de Janeiro, v. 111, n. 3, p. 168-173, 2016.) that validated two ELISA tests produced by Bio-Manguinhos® (Fiocruz, Rio de Janeiro, Brazil), one using crude antigen from *L. major,* currently used as confirmatory test by official LVC diagnostic laboratories, and a second ELISA that tested crude antigen from L. *infantum,* were found for *L. major* sensitivity (Se) of 91.85%, specificity (Sp) 83.75% and accuracy of 0.917, being 89.80% (Se), 82.69% (Sp) and 0.893 accuracy for *L. infantum.* In other words, the ELISA-LPG method of this invention presents more satisfactory standardization values than the test recommended by the Brazilian government.

It is evident that the above examples have been presented for illustrative purposes only, and that modifications and variations of them, obvious to the technicians on the subject, are considered to be included in the scope of the present invention, as defined in the claims below.

## Claims

1. A method to detect antibodies against *Leishmania* in a canine biological sample, the method **characterized by** consisting of the steps:
(i) contacting a biological sample of the said individual with a lipophosphoglycan; and
(ii) detecting the binding of the said lipophosphoglycan with the anti-Leishmania antibody in the biological sample, using a detection agent, thus detecting *Leishmania* infection in the said individual.

2. The method according to claim 1, **characterized by** the fact that the individual is a dog.

3. The method according to claim 2, **characterized by** the fact that the dog is asymptomatic.

4. The method according to claim 1, **characterized by** the fact that the biological sample is selected from the group consisting of blood, serum, plasma, saliva, milk, colostrum, urine, cerebrospinal fluid and tear.

5. The method according to claim 1, **characterized by** the fact that the lipophosphoglycan is bonded, conjugated or immobilized on or to a solid support.

6. The method according to claim 5, **characterized by** the fact that the solid support is a microtiter plate.

7. The method according to claim 1, **characterized by** using a marked detection agent.

8. The method according to claims 7 and 8, **characterized by** the fact that the marked detection agent is an antibody.

9. The method according to claim 7, **characterized by** the fact that the detection agent is marked with a selected marker of an enzyme, a radioisotope, a fluorescent group, a luminescent group, biotin, or dye particles.

10. The method according to claim 7, **characterized by** the fact that it also includes a step of comparing lipophosphoglycane-specific antibody levels with the control level, where the lipophosphoglycane-specific antibody level above the control level is indicative of *Leishmania* infection.

11. A kit for the detection of *Leishmania* infection in a biological sample from an individual, **characterized by** the fact that it includes: the *Leishmania* lipophosphoglycan, a marked detection agent, a set of positive and negative controls, and instructions for use.

12. Use of *Leishmania* lipophosphoglycan, **characterized by** the fact that it is in the manufacture of a kit to detect *Leishmania* in a biological sample from an individual, or in a method to detect antibodies against *Leishmania* in an individual.

## Amended claims

### Amended claims under Art. 19.1 PCT

1. A method to detect antibodies against *Leishmania infantum* in a canine biological sample **characterized by** identifying both symptomatic and asymptomatic dogs comprising the steps:
(i) connect a biological sample of the said individual with a type I lipophosphoglycan of *Leishmania infantum in vitro*; and
(ii) detect the binding of type I lipophosphoglycan with the anti*-Leishmania* antibody in the biological sample, using a detection agent, thus detecting *Leishmania infantum* infection in said individual.

2. The method according to claim 1, **characterized by** the fact that the individual is a dog.

3. The method according to claim 2, **characterized by** the fact that the dog is symptomatic or asymptomatic.

4. The method according to claim 1, **characterized by** the fact that the biological sample is selected from the group consisting of blood, serum, plasma, saliva, milk, colostrum, urine, cerebrospinal fluid and tear.

5. The method according to claim 1, **characterized by** the fact that lipophosphoglycan type I of *L. infantum* is attached, conjugated or immobilized on or to a solid support.

6. The method according to claim 5, **characterized by** the fact that the solid support is a microtiter plate.

7. The method according to claim 1, **characterized by** using a marked detection agent.

8. The method according to claim 7, **characterized by** the fact that the marked detection agent is an antibody.

9. The method according to claims 7 and 8, **characterized by** the fact that the detection agent is marked with a selected marker of an enzyme, a radioisotope, a fluorescent group, a luminescent group, biotin, or dye particles.

10. The method according to claim 1, **characterized by** the fact that it also comprises a step of comparing the levels of antibody specific to a type I lipophosphoglycan molecule with the control level, in which the level of antibody specific to a type I lipophosphoglycan molecule above the control is indicative of infection by *Leishmania infantum.*

11. A kit for detection of *Leishmania infantum* infection in a canine biological sample, **characterized by** the fact that it comprises: a type I lipophosphoglycan molecule of *Leishmania infantum,* a marked detection agent, a set of positive and negative controls.

12. Use of lipophosphoglycan molecule type I of *Leishmania infantum,* **characterized by** the fact that it is in the manufacture of a kit, according to claim 11.

13. Use of a type I lipophosphoglycan molecule of *Leishmania infantum,* **characterized by** the fact that it is in an *in vitro* method, according to claims 1 to 10.
